# EUROPEAN PATENT APPLICATION

(11) **EP 3 764 100 A1**
(43) Date of publication of application: **13.01.2021**
(21) Application number: 19184877.9
(22) Date of filing: 08.07.2019
(51) Int. Cl.: G01N 33/574

(54) **METHOD FOR THE DIAGNOSIS OF OVARIAN CARCINOMA (OC)**

(71) Applicant: Philipps-Universität Marburg, 35037 Marburg (DE)
(72) Inventor: MÜLLER, Rolf, 35041 Marburg (DE); WAGNER, Uwe, 35274 Schönbach (DE)
(74) Representative: Stumpf, Peter

(57) **Abstract**

The invention describes a method and a device for identifying individuals with suspected ovarian carcinoma (OC), especially early stage and relapsed OC, in biological samples from these individuals. The method comprises the steps (i) determining the concentration of suitable markers in a biological sample from an individual, (ii) calculating a multi-marker score, and (iii) comparing the multi-marker score of said individual with a threshold.

## Description

### Technical Field of the Invention

This invention concerns a method for the detection of an ovarian carcinoma and for the monitoring of an ovarian carcinoma recurrence in an individual patient. OC is the common abbreviation of ovarian carcinoma. Early detection means to diagnose OC at an early stage in which no, or only minor clinical symptoms are present, or in which a clear diagnosis is not possible. This invention is therefore also a screening method for the early detection of OC. This invention also enables disease monitoring in patients with already diagnosed and treated OC to allow for the detection of relapsed disease at an early stage providing better therapy options.

OC is a malignant tumour of the ovaries or fallopian tubes (oviducts) in humans or animals, and occurs as solid tumour masses and peritoneal carcinomatosis. OC and OC precursor lesions (e.g. serous tubal intraepithelial carcinoma and cortical inclusion cysts) comprises 2C73.0, 2C74, 2D90, 2D91, and XH8D74 according to ICD-11 classification. OC is usually diagnosed at an advanced stage, since suitable methods for early detection are not available. OC is the deadliest of all gynaecological malignancies and shows an overall 5-year survival rate of less than 50%. The most common form of OC is high-grade serous ovarian cancer (HGSOC). HGSOC makes up the majority of OC cases and has the lowest survival rate.

The peritoneal fluid or ascites fluid is the free fluid in the abdominal and pelvic cavities. In a large number of diseases, peritoneal fluid is increasingly produced, which is then referred to as ascites. The causes of ascites can therefore be manifold. One possible cause of ascites is the growth of a tumour in the abdominal and pelvic cavities. In patients with OC, tumour cells can be shed at a very early stage of the disease and spread within the abdominal and pelvic cavity via the peritoneal fluid to form metastases on the peritoneal wall, in the omentum and the serous membranes of other organs. Even though first-line therapy (surgery and chemotherapy) frequently achieves clinically tumour-free state, most patients relapse due to the outgrowth of residual cancer cells.

Markers (biomarkers) are substrates of the human or animal body or in human or animal body fluids such as blood, serum, plasma, or peritoneal fluid, which are useful for diagnosis of diseases and/or for determination of the prognosis of a disease. Markers are measurable indicators of the presence or severity of a disease. The concentration of markers is determined in order to obtain a diagnosis and/or prognosis. The concentration of markers can be increased or decreased, depending on the marker and the disease. Markers can be proteins, peptides, or oligopeptides; they also can be low molecular mass organic substances or elements.

Biological samples are biological material from living or deceased organisms, which are examined for diagnostic purposes. Biological samples can be for example solid specimens, tissues, cells, or body fluids such as blood, serum, plasma, or peritoneal fluid. Depending on which biological sample is available for the examination of biomarkers, different examination methods are used. Since the concentration of a biomarker in a disease can vary depending on the biological sample used, the selection of the appropriate biological sample is very important. For example, the concentration of biomarkers in OC varies depending on whether blood plasma or peritoneal fluid is used as a biological sample.

### State of the Art

The following known methods can be used for the diagnosis of OC:
- Imaging diagnostics: Ultrasound devices, X-ray systems, magnetic resonance tomographs, computer tomographs or positron emission tomographs can be used for the diagnosis of OC, but are not suitable for the detection of early stages and small tumour masses. The small tumour masses in early stage OC are easily overlooked by imaging diagnostics, especially in peritoneal carcinomatosis or disseminated relapses. For example, a tumour mass smaller than 5 mm in diameter and peritoneal carcinomatosis are usually undetectable by imaging diagnostics. Furthermore, imaging diagnostics are expensive and the results depend on the experience and skill of the examiner. The use of X-rays poses a potential hazard for the patient, in particular for patients with an existing desire to have children. For these reasons, imaging diagnostics are not suitable for the screening for OC as a preventive medical check-up.
- Endoscopic examination: OC can be diagnosed by laparoscopic examination. However, laparoscopy is performed only in unclear symptomatic or otherwise suspicious situations, and is unsuitable for screening purposes or follow-up examinations after first-line therapy. In addition, laparoscopy is expensive and the results depend on the experience and skill of the examiner. Laparoscopy can have adverse sequelae for the patient because of the need to open the peritoneal (abdominal or pelvic) cavity. For these reasons, endoscopic examinations are not suitable for the screening of OC in patients undergoing a preventive medical check-up.
- Histological-pathological examination of tissue samples: This examination can only be carried out if tissue samples of the suspected tumour are available. Taking a tissue sample is an invasive procedure that can be associated with serious complications. Histological-pathological examinations are therefore unsuitable for screening or follow-up examinations or for preventive medical check-ups.
- Blood test: The general parameters such as cell count, differential blood count and clinical-chemical parameters are not meaningful with regard to the presence of early stage OC.
- Determination of the concentration of markers (biomarkers) like transthyretin (TTR), transferrin (TF), apolipoprotein A1 (APOA1), cancer antigen 125 (CA125, MUC-16, mucin 16), human chorionic gonadotropin (HCG), alpha fetoprotein (AFP), lactate dehydrogenase (LDH), carcinoembryonic antigen (CEA), choriogonadotropin-beta (CGB), inhibin B, and human epididymis (epididymal) protein 4 (HE4, WFDC2) in blood plasma: This procedure is unreliable because false negative and/or false positive results often occur. Therefore, the determination of these markers cannot distinguish patients with OC from patients with benign diseases and healthy women with satisfactory certainty. For example, the most widely used marker for the detection of recurrent OC is CA125, but its routine clinical application has not resulted in any substantial improvement in patient survival, since it is neither a sensitive nor a highly specific OC marker. Nevertheless, most physicians use the biomarker CA125, so this marker is currently the gold standard. These markers, like many other biomarkers, are proteins, peptides, or oligopeptides. Their concentration in a biological sample can be measured by different methods: Well-known and established methods are mass spectrometry, ELISA, or electrochemiluminescence immunoassay.

Another method employs the SOMAscan® Proteomic Assay by SomaLogic Inc. The SOMAscan® Proteomic Assay currently measures up to approximately 1,300 markers or protein analytes in a biological sample for instance in serum, plasma, peritoneal fluid or cerebrospinal fluid. The SOMAscan® Proteomic Assay is able to quantify markers that span over eight logs in abundance from femtomolar to micromolar. The SOMAscan® Proteomic Assay uses aptamer technology. The employed aptamers (SOMAmer reagents) have a dual nature as protein affinity-binding reagents with defined three-dimensional structures, and unique nucleotide sequences recognizable by specific DNA hybridization probes. The SOMAscan® Proteomic Assay transforms the protein present in a biological sample into a specific DNA signal. The results of standard DNA quantification techniques are normalized to assure data consistency by correcting for variations due to the SOMAscan® Proteomic Assay, thereby allowing for comparison of samples across a plate. The normalized results obtained for replicate calibration samples included in the assay are then compared to a previous established reference calibration value. This generates a calibration scale factor that is applied to all samples. The final data are reported in relative fluorescent units (RFU) after normalization and calibration. An advantage of the SOMAscan® Proteomic Assay over mass spectrometry is its applicability to unfractionated blood plasma and related fluids like as peritoneal fluid in spite of the high abundance of blood proteins such as albumin, globulins, and fibrinogen, which has been a limiting factor for all previous mass spectrometry-based proteomic analysis of blood plasma.

Another method applies the antibody-based proximity extension assay (PEA) platform by Olink Proteomics (Sweden). PEA uses pairs of oligonucleotide-coupled antibodies binding epitopes in close proximity on the target protein. As a result, the covalently coupled oligonucleotides anneal into a target for proximity-dependent DNA polymerization, subsequently amplified by quantitative polymerase chain reaction (qPCR). A strong advantage of PEA over mass spectrometry (MS) is its applicability to unfractionated plasma and related fluids in spite of the massive dynamic range of protein concentrations caused by analytes such as albumin and globulins.
- Multivariate index assay (MIA): A multivariate index assay for in vitro diagnosis is a method that combines the values of multiple markers using an interpretation function to yield a single, patient-specific result that is indented for use in the diagnosis of disease. For example, an 11-marker panel composed of CA125, CA19-9, EGF-R, C-reactive protein, myoglobin, apolipoprotein A1, apolipoprotein CIII, MIP-1alpha, IL-6, IL-18, and tenascin C was described for the diagnosis of OC. Although one of the currently available MIA shows a sensitivity of 91.3 % and a specificity of 88.5 %, this is not enough to achieve a positive predictive value of 10 % and a low number of false-positive results.

In 2016, the FDA approved Overa® by Vermillion Inc., a test in essence combining two MIAs based on apolipoprotein A-1 (APOA1), CA125, follicle-stimulating hormone (FSH), HE4 and transferrin (TF). The Overa® test used in a diagnostic setting achieves a higher sensitivity and specificity than any individual parameter evaluated to date, and identified 75% of OCs missed by clinical examination alone.
- Genetic tests of liquid biopsies: Some genetic alterations are associated with OC, for example a mutation in the TP53, BRCA1, or BRCA2 genes. However, genetic tests of liquid biopsies have not provided any tools for the detection of early stage OC.

For the diagnosis of recurrences of OC in the context of disease monitoring, determination of the concentration of CA125 in blood plasma is performed: Changes of CA125 in blood plasma concentration should indicate tumour growth or recurrence. However, the concentration of CA125 in blood plasma does not always increase even with proven recurrences, and may be increased in other conditions, such as endometriosis, ovarian cysts, pelvic inflammatory disease, uterine fibroids, leiomyomas, and menstruation. Therefore, both false negative and false positive results are common in this procedure. This is particularly disadvantageous, as in these cases no appropriate therapy is carried out despite existing primary OC or OC recurrences.

A better performance in terms of higher specificity has been observed for Human Epididymal Protein 4 (HE4, also known as WFDC2: WAP Four-Disulfide Core Domain 2), in particular in combination with CA125, but the slight improvement has not resulted in its routine clinical application. Scientists have been trying to overcome the inadequacy of CA125 by combining it with various other markers, but all attempts failed to reliably identify OC at a stage when its detection may affect disease outcome. Likewise, the application of other clinically evaluated biomarkers, including carcinoembryonic antigen (CEA), lactate dehydrogenase (LDH), choriogonadotropin-β (CGB), inhibin B and α-fetoprotein has not improved the situation significantly. For example, a repeated sub-sampling validation was used to identify an optimal combination of markers in a set of eight proteins. Out of these, a 4-marker panel comprising CA125, CA72-4, HE4 and metalloproteinase 7 (MMP7), performed with the highest sensitivity (83.2%) at 98% specificity. However, in view of low prevalence of OC the specificity of this panel is too low to achieve a satisfactory positive predictive value and acceptable level of false-positive results. Another example is the 4-marker panel consisting of CA125, HE4, E-cadherin (CDH1) and IL-6, which performed better than CA125 or HE4 alone or combination of CA125 and HE4.

A number of markers in blood, blood serum, or blood plasma has already been analysed in a non-systematic manner and suggested for the diagnosis of OC, but none of them was validated as a suitable marker for detection of early stage OC. Because of the low prevalence of OC, any screening method for diagnosing OC requires a minimum specificity and sensitivity of >99 % to achieve an acceptable positive predictive value. For the differential diagnosis of suspected or the detection of relapsed OC the requirements in terms of minimum specificity and sensitivity are lower to achieve an acceptable positive predictive value due to the much higher prior probability. For the diagnosis of suspected or relapsed OC the sensitivity is of particular relevance to avoid false negatives (unrecognized cancers or relapses). In this case a minimum specificity and sensitivity of >90 % would be desirable. None of the currently available methods fulfils this criterion. Therefore, there are currently no suitable screening methods for the detection of early stage OC (early detection).

### Problems

There is currently no reliable screening method for the detection of early stage OC with a minimum specificity and sensitivity of >99 % to achieve an acceptable positive predictive value.

There is currently no reliable method for the early detection of relapsed OC.

There is currently no reliable method for the monitoring of patients with OC after therapy especially in patients where the tumour has not been completely removed or has not completely receded. These patients may enter a state of stable disease. In this state, no known tumour marker could give valuable information about the onset of progression.

There is currently no reliable method to test the efficacy of a therapy in patients with OC.

This invention solves the following tasks:
- Improved diagnosis of OC;
- Improved diagnosis of recurrences of already treated OC as a follow-up after therapy;
- Improved diagnosis of early stages of OC;
- Improved control of the efficacy of therapy in patients with OC;
- Improved screening of OC in individuals without any clinical signs.

A serious limitation of all previous efforts for diagnosing OC is the lack of any unbiased global analysis for the discovery of OC markers. Another limitation is the lack of novel biomarkers for diagnosing OC and the lack of reliable scoring systems in the context of OC. Therefore, the inventors used this approach for the present invention.

### Solution

Claim 1 solves the inventive task. The main inventive steps are first the identification of the most significant markers in a biological sample (blood, blood plasma, or blood serum) that have the highest significance to the occurrence or recurrence of OC, and second a biostatistical analysis of the concentration of these markers in patients with OC in comparison with subjects with non-malignant diseases. This invention describes the markers for the diagnosis of OC, especially early stage and relapsed OC. This invention also describes the calculation of multi-marker scores and their use for identifying patients with OC.

Before the inventive method can be performed, the individual (subject, patient) must provide a biological sample (blood, blood plasma, or blood serum). These samples are obtained for example from patients with suspected OC, especially early stage and relapsed OC. These samples are also obtained from any healthy individual for screening purposes, in particular individuals with a family history of breast cancer or OC. Usually these samples are provided by a physician. The collection of blood in order to obtain a biological sample is not stressful for the patient and is a routine medical procedure. The most useful biological sample to apply the invention is blood plasma.

The key feature of the invention is the selection of markers from a very large number of proteins, peptides, or oligopeptides in a biological sample (blood, blood plasma, blood serum, or other non-solid biological tissue) determined by an unbiased analysis that enables a reliable diagnosis of OC, especially early stage and relapsed OC. The inventors identified several markers out of 368 potential markers, which allow for a reliable diagnosis in patients with OC. The inventors found following markers most reliable:
- WFDC2 (synonyms: WAP four-disulphide core domain protein 2, HE4, Human Epididymis Protein 4, EDDM4, WAP5, dJ461P17.6, WAP four-disulphide core domain 2)
- MUC-16 (synonyms: CA125, mucin 16, cancer antigen 125, carcinoma antigen 125, carbohydrate antigen 125)
- SPINT2 (synonyms: Kunitz-type protease inhibitor 2, DIAR3, HAI-2, HAI2, Kop, PB, serine peptidase inhibitor Kunitz type 2, serine peptidase inhibitor Kunitz type 2)
- CHI3L1 (synonyms: Chitinase-3-like protein 1, YKL-40, cartilage glycoprotein-39, ASRT7, CGP-39, GP-39, GP39, HC-gp39, HCGP-3P, YKL40, YYL-40, hCGP-39, chitinase 3 like 1, YK-40)
- N2DL-2 (synonyms: UL16 binding protein 2, ULBP2, RAET1H, ALCAN-alpha, NKG2DL2, RAET1L)
- IL6 (synonyms: Interleukin-6, BSF2, HGF, HSF, IFNB2, IL-6, BSF-2, CDF, IFN-beta-2)
- KLK6 (synonyms: Kallikrein-6, Bssp, Klk7, PRSS18, PRSS9, SP59, hK6, kallikrein related peptidase 6)
- PVRL4 (synonyms: Poliovirus receptor-related 4, NECTIN4, nectin cell adhesion molecule 4, LNIR, PRR4, EDSS1).
- NEP (synonyms: Neprilysin, membrane metallo-endopeptidase (MME), neutral endopeptidase, cluster of differentiation 10, CD10, common acute lymphoblastic leukemia antigen, CALLA, SFE, CMT2T, SCA43)
- hK11 (synonyms: KLK11, human kallikrein-11)

The inventive method for diagnosing OC comprises following steps:
In a first step, the concentration of specific markers in the biological sample of an individual is measured. The concentration of these markers can be measured by ELISA-based techniques, mass spectrometry, antibody- or aptamer-based microarrays or any other suitable quantification method, however preferably by an antibody-based proximity extension assay (PEA).
In a second step, the measured concentration of each marker is normalized. To achieve a normalization of each marker, the measured concentration of each marker was divided by a reference. If the markers are measured by PEA technology the corresponding reference can be seen in Table 3, fourth column from the left (Reference value for normalization (Olink units)). For example, the reference for SPINT2 is 5.31. After division by a reference a multiplication by 100 is performed. The formula for this normalization of the measured concentrations of each marker is therefore: measured concentration / reference * 100.
In a third step, the normalized concentrations of each marker are added to form a multi-marker score. Therefore, the formula for this addition is: Σ normalized concentration of Marker 1 to normalized concentration of Marker x. This sum is the multi-marker score for the measured markers.
In a fourth step, the multi-marker score is compared with a predefined threshold. If the markers WFDC2, MUC-16 and SPINT2 are measured by PEA technology, the predefined threshold is 290.8 normalized units (see Table 3, column far right). If the markers WFDC2, MUC-16, SPINT2, CHI3L1 and N2DL-2 are measured by PEA technology, the predefined threshold is 470.6 normalized units (see Table 3, column far right). If the markers WFDC2, MUC-16, SPINT2, CHI3L1, N2DL-2, IL6, KLK6, PVRL4, NEP and hK11 are measured by PEA technology, the predefined threshold is 1084.4 normalized units (see Table 3, column far right).

It is also possible to measure the concentrations of the first 4, the first 6, the first 7, the first 8 or the first 9 markers listed in Table 3. In these embodiments, the matching predefined thresholds are listed in the corresponding line in Table 3, too. If the multi-marker score is above the predefined threshold, the individual has OC. If the multi-marker score is below the predefined threshold, the individual has no OC.

In case the inventive method is used for the follow-up after therapy, the multi-marker score would probably already be above the predefined threshold due to incomplete removal or incomplete tumour recession. In this case, an increase in the multi-marker score gives valuable information about the onset of progression. This means that the patient is in a state of stable disease if the multi-marker score stays stable. When the multi-marker score starts to rise then it shows a beginning progression of the tumour.

In case the inventive method is used to control the efficacy of the therapy, a decrease in the multi-marker score indicates that the therapy is working. If the multi-marker score does not decrease after the beginning of the therapy, the therapy may not work.

In order to execute the method according to the invention a device (e.g. test kit) can be used. This device is a test kit for determining the concentration of markers in a biological sample like as blood, blood plasma, or blood serum from a subject with suspected OC or with suspected recurrence of OC. This device for diagnosing OC according to claim 7 comprises at least following components:
- A unit for measuring the concentration of at least the markers WFDC2, MUC-16 and SPINT2 in a biological sample of an individual whereby this unit is capable to forward the measured concentration to a data processing unit. Preferably, the unit for measuring is able to measure the concentration of the markers WFDC2, MUC-16, SPINT2, CHI3L1 and N2DL-2. More preferably, the unit for measuring is able to measure the concentration of the markers WFDC2, MUC-16, SPINT2, CHI3L1, N2DL-2, IL6, KLK6, PVRL4, NEP and hK11;
- A data processing unit which receives the measured concentrations from the measuring unit and which executes the steps (ii) to (iv) of claim 1 and forwards the results to the output unit. The data processing unit normalizes the measured concentrations, it calculates a multi-marker score and it compares the multi-marker score with a predefined threshold;
- An input unit for data entry which is connectable to a database for the input of at least the references for normalization and the predefined thresholds and which forwards data to the data processing unit;
- An output unit for data output which indicates OC, if the multi-marker score of step (iii) is higher than the threshold and which indicates no OC, if the multi-marker score of step (iii) is lower than the threshold.

Preferably, the device according to claim 7 utilizes blood plasma as biological sample.

In a preferred embodiment, the concentrations of at least the markers WFDC2, MUC-16 and SPINT2 are determined in step (i) in the biological sample of an individual.

In another preferred embodiment, the concentrations of at least the markers WFDC2, MUC-16, SPINT2, CHI3L1 and N2DL-2 are determined in step (i) in the biological sample of an individual.

In another preferred embodiment, the concentrations of at least the markers WFDC2, MUC-16, SPINT2, CHI3L1, N2DL-2, IL6, KLK6, PVRL4, NEP and hK11 are determined in step (i) in the biological sample of an individual.

The invention also comprises a computer software to perform the method according to claim 1. In a preferred embodiment, this computer software is implemented in the data processing unit and performs the steps (ii), (iii) and (iv) of claim 1.

### Embodiments

In order to identify the most significant markers for OC the inventors determined the concentration of 368 potential markers in blood plasma samples of 20 patients with proven OC (OC-plasma) and in blood plasma samples of 20 subjects with non-malignant diseases (N-plasma) by the Proximity Extension Assay (PEA) offered by Olink Proteomics (Sweden).

The concentrations of at least three markers listed in Table 1 in conjunction with suitable biostatistical analyses are used to predict the presence of OC in an individual. In a preferred embodiment, a multi-marker score is calculated as follows: PEA units for markers identified as significantly upregulated in OC-plasma versus N-plasma controls are median-normalized marker-wise. Within this set, the marker yielding maximum separation between the lowest level in OC-plasma and the highest level in N-plasma samples is identified. This marker is WFDC2. Iterating over the median-centered values of the remaining upregulated probe signals and adding their values individually to the respective previous sum (starting with the WFDC2 measurement), the protein maximally increasing the OC- to N-plasma distance is identified. This procedure is repeated to obtain sequentially larger combinations, until all significant markers are included.

### 1. Determination of markers in biological samples like blood, blood plasma, or blood serum as markers for OC

Since biological samples contain a large number of proteins in different concentrations, it is very important to measure the most suitable biological sample with the method suitable for the biological marker to be determined.

Extensive investigations and extensive biostatistical analyses of the inventors have shown that there is a high correlation between clinical findings and the concentration of certain markers in blood, blood plasma or blood serum of patients with OC. Since most markers of interest in OC-patients are presumably proteins, a proteomics-based global analysis of blood plasma was performed in patients with proven OC (OC-patients, OC-plasma) and in subjects with non-malignant diseases (N-patients, N-plasma). Subjects with non-malignant diseases had ovarian cysts or myoma uteri. In order to perform this proteome analysis the antibody-based Proximity Extension Assay (PEA) offered by Olink Proteomics (Sweden) was utilized. The data from the proteome analysis were then biostatistically evaluated.

For proteome analysis, 20 samples of blood plasma were examined from patients with high-grade serous OC (OC-plasma). For comparison, 20 samples of blood plasma from subjects with non-malignant diseases were examined (N-plasma). The concentration of 368 markers was determined by PEA in these samples.

The inventive step is to select from these 368 markers in the blood plasma those combinations that have a highly significant correlation with the presence of OC. Biostatistical analyses identified significantly higher concentrations in OC-plasma versus N-plasma for 157 marker proteins (see Table 1). The indicated and adjusted p-values were determined by Mann-Whitney U test and adjusted for multiple hypothesis testing by Benjamini-Hochberg correction. The rank order reflects the increasing adjusted p-value.

**Table 1**

| **Marker** | **Fold change (OC/N)** | **Adjusted p-value** |
|---|---|---|
| MUC-16 | 5.29 | 6.80E-06 |
| IL6 | 3.93 | 6.80E-06 |
| CHI3L1 | 2.76 | 6.80E-06 |
| SPINT2 | 2.26 | 6.80E-06 |
| PVRL4 | 2.03 | 6.80E-06 |
| WFDC2 | 1.96 | 6.80E-06 |
| N2DL-2 | 1.62 | 6.80E-06 |
| WISP-1 | 1.61 | 6.80E-06 |
| KLK6 | 3.63 | 8.99E-06 |
| SPON1 | 3.11 | 8.99E-06 |
| hK11 | 1.66 | 8.99E-06 |
| DDR1 | 1.35 | 8.99E-06 |
| VEGFA | 1.24 | 9.49E-06 |
| OPN | 1.59 | 1.01E-05 |
| BCAM | 1.68 | 1.08E-05 |
| SPINT1 | 2.99 | 1.50E-05 |
| CDH6 | 1.51 | 3.06E-05 |
| VSIG4 | 1.68 | 3.53E-05 |
| GFR-alpha-1 | 1.41 | 3.53E-05 |
| IGFBP-2 | 1.54 | 3.62E-05 |
| TFPI-2 | 1.21 | 3.62E-05 |
| FLRT2 | 1.53 | 4.43E-05 |
| TNFRSF12A | 1.56 | 6.13E-05 |
| EDA2R | 1.80 | 6.64E-05 |
| FUT3/FUT5 | 2.16 | 6.92E-05 |
| TNFRSF6B | 1.54 | 6.92E-05 |
| AREG | 1.64 | 7.51 E-05 |
| PTPRF | 1.62 | 7.89E-05 |
| GDF15 | 1.62 | 7.89E-05 |
| COL1A1 | 1.69 | 8.59E-05 |
| B4GALT1 | 1.64 | 9.35E-05 |
| IL2-RA | 1.73 | 1.02E-04 |
| EFNA4 | 1.77 | 1.40E-04 |
| MSLN | 2.75 | 1.48E-04 |
| EPHA2 | 1.67 | 1.48E-04 |
| ADAM-TS 15 | 1.35 | 1.62E-04 |
| FCRLB | 1.92 | 1.72E-04 |
| vWF | 1.31 | 1.72E-04 |
| DSC2 | 1.67 | 1.83E-04 |
| CRHBP | 1.32 | 1.83E-04 |
| SYND1 | 1.40 | 1.90E-04 |
| TNF-R1 | 1.33 | 1.90E-04 |
| HGF | 1.29 | 1.90E-04 |
| LAYN | 1.59 | 2.54E-04 |
| U-PAR | 1.53 | 2.54E-04 |
| PLXNB1 | 2.33 | 2.77E-04 |
| ANGPTL4 | 1.47 | 2.90E-04 |
| TNFRSF21 | 1.18 | 2.90E-04 |
| FURIN | 1.17 | 2.90E-04 |
| NRP2 | 1.36 | 3.17E-04 |
| TNFSF13 | 1.20 | 3.85E-04 |
| MK | 2.28 | 4.13E-04 |
| HAVCR2 | 1.74 | 4.13E-04 |
| GPC1 | 1.31 | 4.50E-04 |
| FSTL3 | 1.59 | 4.91 E-04 |
| KLK13 | 1.54 | 5.36E-04 |
| DRAXIN | 3.16 | 5.74E-04 |
| S100A11 | 1.37 | 5.74E-04 |
| SCARB2 | 1.54 | 6.26E-04 |
| TGF-alpha | 1.70 | 7.43E-04 |
| LTBR | 1.54 | 7.97E-04 |
| RELT | 1.32 | 7.97E-04 |
| IGF1R | 1.44 | 8.67E-04 |
| ST2 | 1.69 | 1.23E-03 |
| TNFRSF4 | 1.59 | 1.23E-03 |
| CTSD | 1.64 | 1.30E-03 |
| LAIR1 | 1.50 | 1.30E-03 |
| FABP4 | 1.48 | 1.30E-03 |
| EPHB4 | 1.29 | 1.41E-03 |
| PODXL | 1.39 | 1.54E-03 |
| PDGF-R-alpha | 1.33 | 1.64E-03 |
| EPHB6 | 1.42 | 1.76E-03 |
| NBL1 | 1.17 | 1.76E-03 |
| CD74 | 4.34 | 1.91 E-03 |
| DAG1 | 1.23 | 2.07E-03 |
| Alpha-2-MRAP | 1.21 | 2.70E-03 |
| hK8 | 1.23 | 2.88E-03 |
| CD27 | 1.18 | 2.88E-03 |
| CLM-1 | 1.28 | 3.12E-03 |
| HTRA2 | 1.76 | 3.64E-03 |
| TMSB10 | 2.13 | 3.89E-03 |
| SPINK1 | 1.61 | 3.89E-03 |
| TNF-R2 | 1.30 | 4.20E-03 |
| CSTB | 1.61 | 5.33E-03 |
| MMP-9 | 1.51 | 5.74E-03 |
| ERBB4 | 1.28 | 6.18E-03 |
| NT-proBNP | 2.43 | 6.44E-03 |
| TIMP4 | 1.53 | 6.44E-03 |
| SMOC2 | 1.19 | 6.44E-03 |
| ESM-1 | 1.14 | 6.44E-03 |
| TNFRSF19 | 1.34 | 7.23E-03 |
| IGFBP-1 | 1.28 | 7.23E-03 |
| CXCL16 | 1.21 | 7.23E-03 |
| MAD homolog 5 | 1.53 | 7.70E-03 |
| CCL15 | 1.19 | 7.70E-03 |
| GP6 | 1.42 | 8.27E-03 |
| PILRA | 1.45 | 8.80E-03 |
| CTSC | 1.26 | 8.80E-03 |
| COLEC12 | 1.27 | 9.36E-03 |
| CLEC11A | 1.18 | 1.00E-02 |
| CADM3 | 2.42 | 1.06E-02 |
| MIC-A/B | 1.28 | 1.06E-02 |
| VEGFR-3 | 1.05 | 1.06E-02 |
| CRELD2 | 1.41 | 1.11E-02 |
| IL-5R-alpha | 1.34 | 1.11E-02 |
| MSR1 | 1.26 | 1.11 E-02 |
| SMPD1 | 1.25 | 1.11E-02 |
| JAM-A | 1.28 | 1.36E-02 |
| SIGLEC1 | 1.15 | 1.36E-02 |
| RSPO1 | 1.48 | 1.43E-02 |
| CLEC14A | 1.20 | 1.43E-02 |
| Gal-1 | 1.10 | 1.43E-02 |
| SCARF2 | 1.19 | 1.64E-02 |
| TGFR-2 | 1.16 | 1.64E-02 |
| GDNF | 4.48 | 1.71E-02 |
| PRTN3 | 1.30 | 1.71 E-02 |
| ADAM 8 | 1.14 | 1.71E-02 |
| DLL1 | 1.08 | 1.71 E-02 |
| SHPS-1 | 1.24 | 1.78E-02 |
| IL-1 RT1 | 1.17 | 1.78E-02 |
| t-PA | 1.17 | 1.78E-02 |
| FADD | 1.92 | 1.97E-02 |
| CD38 | 1.21 | 1.97E-02 |
| SCARF1 | 1.16 | 1.97E-02 |
| IFN-gamma-R1 | 1.14 | 1.97E-02 |
| CDH3 | 1.10 | 1.97E-02 |
| CD177 | 1.40 | 2.10E-02 |
| FAS | 1.23 | 2.24E-02 |
| AZU1 | 1.57 | 2.35E-02 |
| Beta-NGF | 1.15 | 2.35E-02 |
| OPG | 1.14 | 2.35E-02 |
| NOV | 1.43 | 2.47E-02 |
| CRIM1 | 1.32 | 2.47E-02 |
| CLM-6 | 1.14 | 2.47E-02 |
| PVR | 1.13 | 2.63E-02 |
| TR | 1.37 | 2.76E-02 |
| TR-AP | 1.28 | 2.76E-02 |
| MMP-3 | 1.21 | 2.76E-02 |
| NUDT5 | 1.28 | 2.94E-02 |
| ESAM | 1.16 | 3.08E-02 |
| PLXNB3 | 1.11 | 3.08E-02 |
| P4HB | 1.84 | 3.21E-02 |
| PPY | 1.16 | 3.21 E-02 |
| RETN | 1.13 | 3.21E-02 |
| NEP | 1.69 | 3.34E-02 |
| IL-18BP | 1.15 | 3.34E-02 |
| SKR3 | 1.07 | 3.34E-02 |
| MATN2 | 1.15 | 3.53E-02 |
| PGLYRP1 | 1.18 | 3.70E-02 |
| CLEC1B | 1.09 | 3.70E-02 |
| TNFSF13B | 1.11 | 3.90E-02 |
| CTSZ | 1.08 | 4.10E-02 |
| sFRP-3 | 2.60 | 4.27E-02 |
| VIM | 1.23 | 4.27E-02 |
| NID2 | 1.10 | 4.27E-02 |
| CTSS | 1.07 | 4.27E-02 |
| TXLNA | 1.16 | 4.52E-02 |

### 2. Functions of upregulated markers

Functional annotation of the markers upregulated in OC plasma by gene ontology (GO) term enrichment analysis identified several biological processes known to be critical for OC growth and progression, including immune regulation, cell adhesion, cell migration, cell proliferation, cell death and extracellular matrix organization. The most significant molecular functions associated with upregulated plasma proteins were membrane-receptor-driven pathways triggered by interactions with extracellular matrix (ECM) components and growth factors, such as IGF (insulin-like growth factor), VEGF (vascular endothelial growth factor), TNF (tumor necrosis factor), semaphorins and PDGF (platelet-derived growth factor), as well as extracellular proteases and their inhibitors. These findings are consistent with the current knowledge of progression-driving mechanisms in OC.

### 3. Correlation of PEA, SOMAscan® Proteomic Assay and ELISA data

To assess the validity of the results obtained by PEA (antibody-based Olink Proteomic platform) all samples were reanalyzed by the aptamer-based SOMAscan® Proteomic Assay using the 1.3k panel with 1,305 probes. Out of the 157 proteins identified by PEA as upregulated in OC-plasma (see above) 107 were present (by gene names) in the SOMAscan® panel. Spearman analysis across all plasma samples revealed a high positive median correlation of rho=0.62 for these 107 proteins between the platforms. As several relevant markers are not part of the SOMAscan® panel (such as MUC-16 and WFDC2), the inventors also determined plasma levels for 12 proteins of the PEA dataset by ELISA. These are BCAM, EPHA2, GDF15, IL6, IL18BP, MUC-16, OPN, PVRL4, SPINT2, SPON1, VEGFA and WFDC2. ELISA could reproduce significant differences between the two plasma sample sets observed by PEA for all markers except EPHA2 (ephrin receptor A2). Consistently, a good correlation (Spearman p>0.5) for 10 of these 12 markers between PEA and ELISA (Table 2) was seen. Of note, an excellent correlation was observed for SPINT2 (p=0.87).

**Table 2**

| **Marker** | **Spearman rho (p)** |
|---|---|
| WFDC2 | 0.96 |
| IL6 | 0.91 |
| SPINT2 | 0.87 |
| MUC-16 | 0.85 |
| PVRL4 | 0.79 |
| OPN | 0.78 |
| BCAM | 0.74 |
| GDF15 | 0.66 |
| IL-18BP | 0.65 |
| VEGFA | 0.54 |
| SPON1 | 0.37 |
| EPHA2 | 0.06 |

### 4. Biostatistical evaluation of proteome analysis and calculation of multi-marker scores

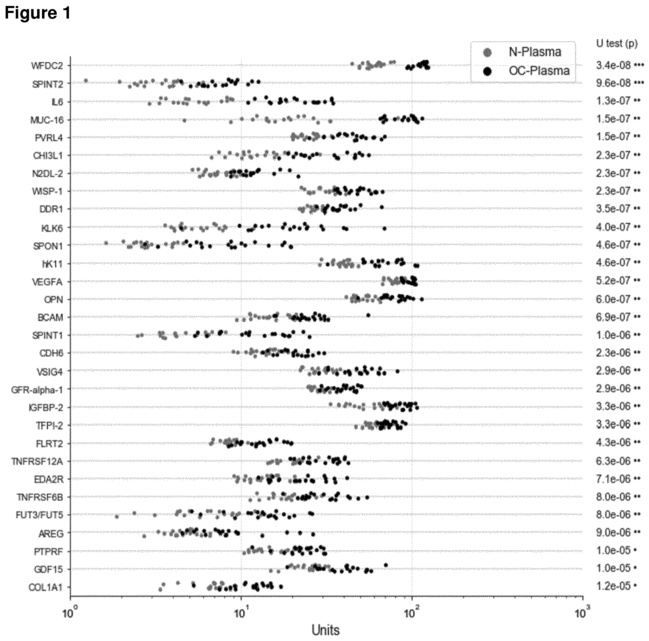

First the inventors identified 157 markers with higher median levels in OC-plasma compared to N-plasma (p-value <0.05 by Mann-Whitney U test; adjusted for multiple hypothesis testing by Benjamini-Hochberg correction; see Table 1). Figure 1 is a graphical representation of the data for the top 30 of these upregulated markers, which shows that except for WFDC2 none of the markers on its own is able to achieve a complete separation of all OC-plasma and all N-plasma samples, which also applies to the clinically used marker MUC-16. Furthermore, the distance between the two sample sets is relatively small for WFDC2, which may explain its inadequacy as a reliable marker observed in previous studies.

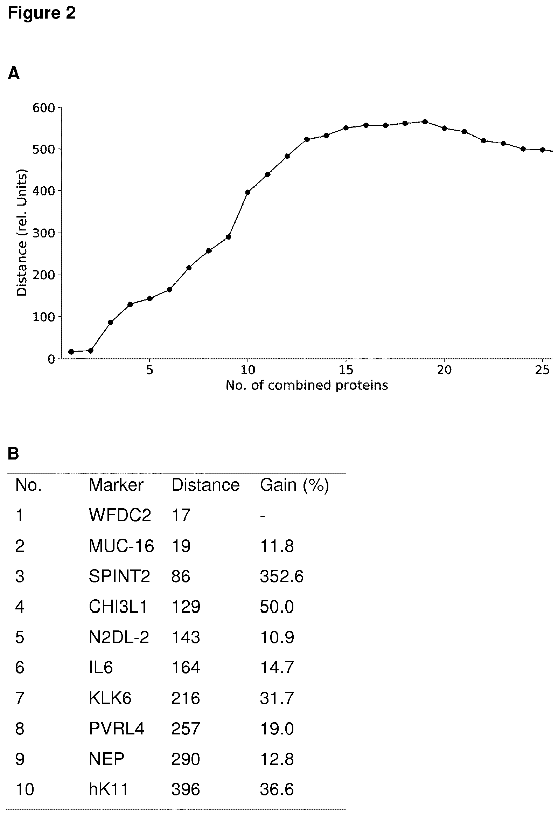

In an attempt to identify multi-marker panels that perform better that the clinically evaluated MUC-16 and WFDC2 markers alone, the inventors tested all possible combinations of the significantly upregulated markers from Fig. 1 for their potential to separate the two groups. To calculate a multi-marker score normalized (median-centered) PEA signals were added up for each patient and the difference between the lowest OC-plasma values and the highest N-plasma value was determined. As expected from the data in Fig. 1, the marker yielding the best separation was WFDC2 (Fig. 2A and B; No. 1). The performance was marginally improved by adding MUC-16 (11.8% gain in distance; Table 3; Fig. 2A and B). The marker improving performance of the WFDC2- MUC-16-combination surprisingly best was SPINT2, which gave rise to a 352% gain. Addition of further markers continuously increased the distance between the two groups of plasma samples in increments of 13-50% for each marker up to a total number of approximately 12 (Fig. 2A and B; Table 3). The inclusion of additional markers led to a marginal gain, and more than 20 markers reduced performance of the panel (Fig. 1; Table 3). Table 3 shows the markers No. 1-19 that can be used for the clear distinction between patients with OC (OC-plasma) and subjects with non-malignant diseases (N-plasma). "Distance" (see third column from the left) indicates the difference of the multi-marker scores between OC-plasma and N-plasma after the measured concentrations were normalized using the indicated reference values (see fourth column from the left) and added up.

The numbers (No.) refer to the rank of the respective marker in the combinations in Figure 2 (e.g., the combination of 10 markers contains marker 1-10 listed in Table 3). The fourth column from the left shows the reference (median of marker concentrations in all plasma samples) used for marker-wise median-centred normalization of measured concentrations (measured concentration / reference * 100) in Figure 2. The column most right shows the predefined thresholds which are used for step (iv) in claim 1.

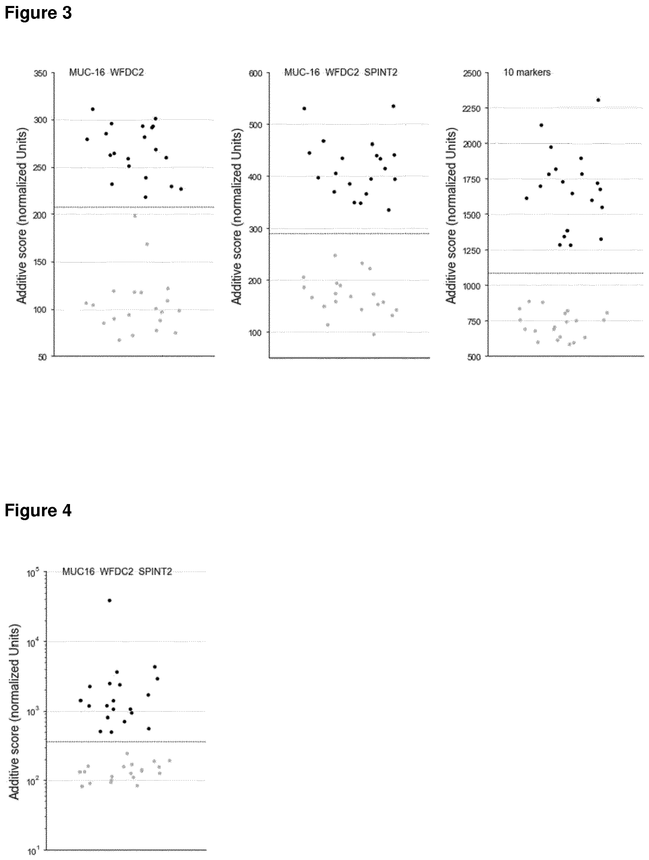

The results are illustrated in detail in Fig. 3. Separation of the two plasma sets was improved by adding SPINT2 to MUC-16 and WFDC2, and was further increased when the 10 best performing proteins form Fig. 2A and B were included. Importantly, the inventors were able to reproduce the performance of the MUC-16 + WFDC2 + SPINT2 combination by an independent ELISA-based measurement of the same sample set (Fig. 4). A clear separation of OC-plasma and N-plasma samples was also achieved when WFDC2 and MUC-16 were omitted from this panel, attesting to the power of proteins other than WFDC2 and MUC-16 identified in the present study to discriminate the two cohorts.

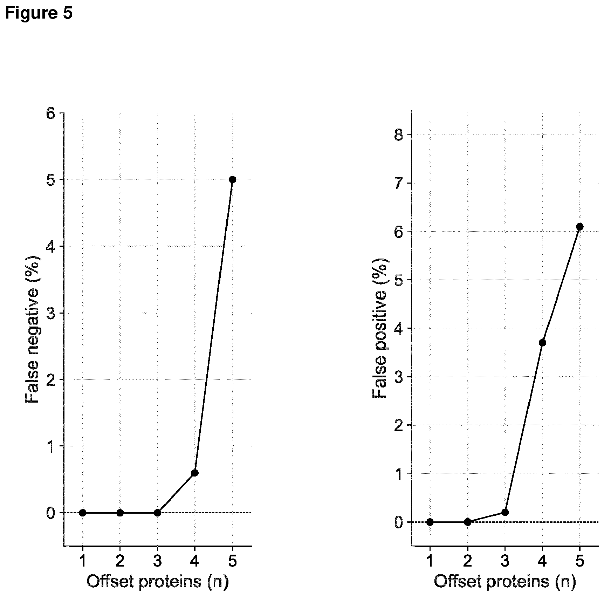

To test the robustness of these multi-marker combinations simulations were performed where the levels of one or more randomly selected marker(s) for one random patient with "outlier" values were changed (i.e., for N-plasma samples with the median of OC-plasma and vice versa). These analyses revealed that for the three-marker combination of WFDC2, MUC-16 and SPINT2 one outlier resulted in false-positives or false-negatives in 3% of the cases. In contrast, the ten-marker combination (WFDC2, MUC-16, SPINT2, CHI3L1, N2DL-2, IL6, PVRL4, NEP, KLK 6 and hK11) tolerated 2 outliers in either the OC-plasma samples (Fig. 5, left; 0/1000 false negatives) of the N-plasma samples (Fig. 5, right; 0/1000 false positives). The same result was obtained when outliers within the 10-marker panel were limited to MUC-16, WFDC2 and SPINT2 (maximum two outliers). These findings indicate a gain in robustness to outliers correlating with the number of markers in multi-marker combinations and thus an advantage of extending panels beyond WFDC2, MUC-16 and/or SPINT2 as the strongest markers identified in the present study.

**Table 3**

| **No.** | **Marker** | **Distance** | **Reference value for normalization (Olink units)** | **Rel. gain (%)** | **Threshold (normalized units)** |
|---|---|---|---|---|---|
| 1 | WFDC2 | 17.6 | 86.53 | N/A | 100.0 |
| 2 | MUC-16 | 19.4 | 68.17 | 9.8 | 208.1 |
| 3 | SPINT2 | 86.9 | 5.31 | 348.1 | 290.8 |
| 4 | CHI3L1 | 129.4 | 18.99 | 49.0 | 370.8 |
| 5 | N2DL-2 | 144.0 | 9.12 | 11.2 | 470.6 |
| 6 | IL6 | 164.0 | 11.57 | 14.0 | 556.1 |
| 7 | KLK6 | 216.4 | 8.99 | 31.9 | 653.1 |
| 8 | PVRL4 | 257.3 | 29.55 | 18.9 | 770.1 |
| 9 | NEP | 290.7 | 3.64 | 13.0 | 964.3 |
| 10 | hK11 | 396.1 | 46.99 | 36.3 | 1084.4 |
| 11 | AREG | 439.0 | 5.71 | 10.8 | 1187.4 |
| 12 | MK | 483.3 | 32.01 | 10.1 | 1309.5 |
| 13 | PTPRF | 524.3 | 18.58 | 8.5 | 1400.7 |
| 14 | WISP-1 | 533.8 | 36.98 | 1.8 | 1507.4 |
| 15 | SPINT1 | 551.5 | 6.65 | 3.3 | 1611.6 |
| 16 | BCAM | 557.2 | 20.28 | 1.0 | 1710.1 |
| 17 | LAYN | 557.3 | 18.67 | 0.0 | 1826.4 |
| 18 | SCARB2 | 562.5 | 11.48 | 0.9 | 1941.5 |
| 19 | CDH6 | 566.2 | 16.12 | 0.7 | 2047.2 |
| 20 | IGFBP-2 | 550.8 | 74.16 | -2.7 | 2149.0 |
| 21 | EPHA2 | 542.2 | 7.99 | -1.6 | 2246.3 |
| 22 | FUT3/FUT5 | 522.0 | 9.36 | -3.7 | 2374.4 |
| 23 | TNFRSF12A | 514.0 | 23.03 | -1.5 | 2485.3 |
| 24 | PLXNB1 | 500.7 | 3.11 | -2.6 | 2593.7 |
| 25 | TNFRSF6B | 498.6 | 23.31 | -0.4 | 2697.6 |

### 5. Methods for determining the concentration of markers

The concentrations of the markers in a biological sample such as blood, blood plasma, or blood serum obtained from a subject with suspected primary or relapsed OC are measured preferably by the antibody-based PEA. Alternatively, the concentrations of these markers can be measured by using other methods like ELISA-based techniques, mass spectrometry, other antibody- or aptamer-based microarrays or any other suitable quantification method. Blood plasma is the most suitable biological sample for the inventive method.

### Description of the Figures

**Figure 1** shows a graphical representation of the data for the top 30 significantly upregulated markers. Black: OC-Plasma; gray: N-plasma.
**Figure 2** illustrates the separation of plasma samples from patients with OC (OC-plasma) and from subjects with non-malignant diseases (N-plasma) by a multi-marker score system consisting of adding up marker-wise normalized PEA units. The graph in panel A shows the distance (i.e. difference) between the minimum of all OC-plasma samples and the maximum of all N-plasma samples in relation to the number of combined markers. For example, the number 10 on the x-axis represents the combination of the first ten markers of Table 3 and panel B. Gain in panel B is the % increase in distance resulting from inclusion of the respective marker.
**Figure 3** shows the distribution in OC-plasma and N-plasma samples for the top 2, 3 and 10 markers in Table 2 and Figure 2. Units means the sum of marker-wise normalized PEA units as in Figure 1 and is the multi-marker score for patients with OC (OC-plasma) and subjects with non-malignant disease (N-plasma). Dots represent individual values. Black: OC-Plasma; gray: N-plasma.
**Figure 4** shows the distribution in OC-plasma and N-plasma samples for the top 3 markers in Table 2 and Figure 2. Units means the sum of marker-wise normalized ELISA concentrations and is the multi-marker score for patients with OC (OC-plasma) and subjects with non-malignant disease (N-plasma). Dots represent individual values. Black: OC-Plasma; gray: N-plasma.
**Figure 5** shows a simulation of sample sets by changing the levels for one or more randomly selected marker(s) for one random patient in the OC-plasma set with "outlier" values 1000-times (i.e., with the median of N-plasma; left panel). The right panel shows an analogous simulation for the N-plasma set (replacement with the median of OC-plasma).

## Claims

1. A method for diagnosing ovarian carcinoma (OC) in a biological sample from an individual with suspected OC or suspected relapsed OC, comprising the steps
(i) measuring the concentration of at least the markers WFDC2, MUC-16 and SPINT2 in the biological sample of said individual;
(ii) normalizing the measured concentrations of each marker from step (i);
(iii) adding the normalized concentrations of each marker from step (ii) to form a multi-marker score;
(iv) comparing the multi-marker score of step (iii) with a predefined threshold;
where the said individual has OC, if the multi-marker score of step (iii) is higher than the threshold and where the individual has no OC, if the multi-marker score of step (iii) is lower than the threshold.

2. A method for diagnosing ovarian carcinoma (OC) in a biological sample from an individual with suspected OC or suspected relapsed OC, according to claim 1, **characterized in that** in step (i) the concentrations of at least the markers WFDC2, MUC-16, SPINT2, CHI3L1 and N2DL-2 are measured.

3. A method for diagnosing ovarian carcinoma (OC) in a biological sample from an individual with suspected OC or suspected relapsed OC, according to claim 1, **characterized in that** in step (i) the concentrations of at least the markers WFDC2, MUC-16, SPINT2, CHI3L1, N2DL-2, IL6, KLK6, PVRL4, NEP and hK11 are measured.

4. A method for diagnosing ovarian carcinoma (OC) in a biological sample from an individual with suspected OC or suspected relapsed OC, according to the previous claims 1 to 3, **characterized in that** the biological sample is blood plasma.

5. A method for diagnosing ovarian carcinoma (OC) in a biological sample from an individual with suspected OC or suspected relapsed OC according to the claims 1 to 4, **characterized in that** the determining of the concentration of said markers is performed by an antibody-based proximity extension assay (PEA).

6. A method for diagnosing OC in a biological sample from an individual with suspected OC or suspected relapsed OC according to the claims 1 to 4, **characterized in that** the determining of the concentration of said markers is performed by ELISA-based techniques, mass spectrometry, antibody- or aptamer-based microarrays, or any other suitable quantification method.

7. A device for diagnosing ovarian carcinoma (OC) in a biological sample from an individual according to claim 1, **characterized in that** the device comprises at least
- a measuring unit for measuring the concentration of at least the markers WFDC2, MUC-16 and SPINT2 in the biological sample of said individual whereby this unit is capable to forward the measured concentration to a data processing unit;
- a data processing unit which receives the measured concentration from the measuring unit and which executes the steps (ii) to (iv) of claim 1 and forwards the results to the output unit;
- an input unit for data entry which is connectable to a database for the input of at least the predefined thresholds and which forwards data to the data processing unit;
- an output unit for data output which indicates OC, if the multi-marker score of step (iii) is higher than the threshold and which indicates no OC, if the multi-marker score of step (iii) is lower than the threshold.

8. Computer software to execute the method according to one of the claims 1 to 6.
